# EUROPÄISCHE PATENTANMELDUNG

(11) **EP 1 164 119 A2**
(43) Veröffentlichungstag der Anmeldung: **19.12.2001**
(21) Anmeldenummer: 01111613.4
(22) Anmeldetag: 12.05.2001
(51) Int. Cl.: C07C 45/73, C07C 49/04, B01J 23/58, B01J 23/63

(54) **Verfahren zur Herstellung von höheren Ketonen aus ungesättigten Aldehyden**

(30) Priorität: 15.06.2000 DE 10028800
(71) Anmelder: BASF AKTIENGESELLSCHAFT, 67056 Ludwigshafen (DE)
(72) Erfinder: Kramer, Andreas, Dr., 67098 Bad Dürkheim (DE); Siegel, Wolfgang, Dr., 67117 Limburgerhof (DE)

(57) **Zusammenfassung**

Die vorliegende Erfindung betrifft ein Verfahren zur Herstellung von höheren Ketonen durch Umsetzung von ungesättigten Aldehyden mit Ketonen in Gegenwart von Wasserstoff und einem Katalysatorsystem enthaltend mindestens eine zur Übertragung von Wasserstoff auf Doppelbindungen fähige Komponente, sowie Oxide der Erdalkalimetalle, der Seltenen Erden oder Gemischen davon.

## Beschreibung

Die vorliegende Erfindung betrifft ein Verfahren zur Herstellung von höheren Ketonen durch Umsetzung von ungesättigten Aldehyden mit Ketonen in Gegenwart von Wasserstoff und einem Katalysatorsystem enthaltend mindestens eine zur Übertragung von Wasserstoff auf Doppelbindungen fähige Komponente, sowie Oxide der Erdalkalimetalle, der Seltenen Erden oder Gemischen davon.

Insbesondere wird das erfindungsgemäße Verfahren verwendet zur Herstellung von Tetrahydrogeranylaceton (6,10-Dimethylundecan-2-on) durch gekreuzte Aldolkondensation unter hydrierenden Bedingungen aus Aceton und einem ungesättigten Aldehyd insbesondere Citral oder Citronellal.

Die Verwendung der Aldolreaktion zum Aufbau höherer Ketone oder Aldehyde ist in der organischen Synthese weit verbreitet und wird in der wissenschaftlichen Literatur umfangreich dokumentiert (Houben-Weyl, Methoden der organischen Chemie, Band 7/1, 4. Auflage, 1979, S. 77 ff.; Band 7/2b S. 1449 ff.).

Neben säurekatalysierten Verfahren sind Methoden zur Aldolkondensation unter Basenkatalyse bekannt, wobei vielfach die Hydroxide der Alkali- und Erdalkalimetalle eingesetzt werden.

Nachteil der Aldolkondensation, insbesondere dann, wenn die Aldolkondensation beispielsweise zwischen einem Keton und einem Aldehyd ausgeführt wird (gekreuzte Aldolkondensation) ist die oft schwer zu lenkende Selektivität, da in diesen Fällen die Reaktion der Reaktanden mit sich selber in größerem Umfang geschieht. Dieses Problem kann oftmals durch die gezielte Herstellung eines Enamins von einer der Reaktionskomponenten gelöst werden. Allerdings wird dort die höhere Selektivität durch die Einführung einer weiteren Synthesestufe und aufwendigen Operationen zur Aufarbeitung erreicht.

Daneben ist auch die Ausführung der gekreuzten Aldolkondensation zwischen Alkanalen und Alkanonen unter hydrierenden Bedingungen an Festkörpern bekannt (DE 262 554 1). Als Aldolisierungskatalysator wir hierbei Zinkoxid in Verbindung mit hydrieraktiven Komponenten wie Nickel und/oder Cobalt verwendet. Auch die Kombination aus einem Oxid der Seltenen Erdmetalle und einem Edelmetall wie Palladium oder Platin kann für die gekreuzte Aldolkondensation unter gleichzeitiger Hydrierung der durch die Aldolkondensation entstehenden Doppelbindung eingesetzt werden (DE 26 15 308).

Die gekreuzte Aldolkondensation zur Herstellung von höheren Ketonen mit ungesättigten Aldehyden führt in der Regel zu Eigenkondensationen, Cyclisierungen oder Polymerisationen.

Es ist bekannt, dass in einer aufwendigen Reaktionsführung die Kondensation von Citral mit Aceton zu Pseudojonon in Gegenwart von Natriumethylat bei -5°C mit einer nur mäßigen Ausbeute von 45 bis 49 % verläuft (Org. Synthesis Coll. Vol. III, 747).

Wird Citral mit alkoholischer Kalilauge behandelt tritt Polymerisation ein (Bull. Soc. Chim. France, Bd. 21, 407) oder Zersetzung zu 6-Methylheptanon und Acetaldehyd, bei Einwirkung von wässriger Alkali auf Citral (Bull. Soc. Chim. France, Bd. 17, 175).

DE 215 099 2 offenbart eine Aldolkondensation von Citral mit Aceton bei 200°C und 80 bar zur Herstellung von Pseudojonon an ZnO-haltigen Katalysatoren, mit einem Umsatz von Citral an 82 % und einer Ausbeute von 88 %. Dort wird ebenfalls offenbart, dass der Einsatz von MgO als Bestandteil des Katalysators zur Eigenkondensation der Aldehydkomponente führt.

Da zahlreiche bei der Kondensation von ungesättigten Aldehyden mit Ketonen unter hydrierenden Bedingungen erhältliche Reaktionsprodukte von großem Interesse sind, beispielsweise Tetrahydrogeranylaceton, als Zwischenstufe in der Synthese von Isophytol und Vitamin E, war es die Aufgabe der Erfindung ein wirtschaftliches Herstellverfahren zu entwickeln, dass die im Stand der Technik beschriebenen Nachteile nicht aufweist und zu hohen Selektivitäten und Ausbeuten führt.

Überraschenderweise wurde nun gefunden, dass sich ungesättigte Aldehyde in einer gekreuzten Aldolkondensation zusammen mit einem Keton, in hohen Ausbeuten und Selektivitäten an festen edelmetallhaltigen Aldolisierungskatalysatoren, die zur Aldolisierung Oxide der Seltenen Erden, der Erdalkalimetalle oder Gemische davon enthalten, in Gegenwart mindestens einer zur Übertragung von Wasserstoff auf Doppelbindungen fähigen Komponente, zu höheren Ketonen in einer wasserstoffhaltigen Atmosphäre umsetzen lassen.

Gegenstand der Erfindung ist ein Verfahren zur Herstellung von höheren Ketonen durch Umsetzung eines Aldehyds der allgemeinen Formel I 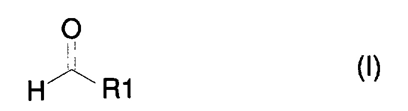 wobei
R¹ einen gegebenenfalls substituierten geradkettigen oder verzweigten ein- oder mehrfach ungesättigten C₁-C₂₀-Alkylrest oder einen gegebenenfalls substituierten C₃-C₇-Cycloalkenylrest bedeutet
   mit einem Keton der allgemeinen Formel II 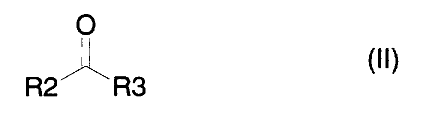 wobei
R², R³ einen gegebenenfalls substituierten geradkettigen oder verzweigten gesättigten oder ungesättigten C₁-C₂₀-Alkylrest oder einen gegebenenfalls substituierten C₃-C₇-Cycloalkyl-, oder Cycloalkenylrest bedeuten oder R² und R³ zusammen einen 3-bis 7-gliedrigen, gegebenenfalls substituierten Carbocyclus bedeuten, in Gegenwart von Wasserstoff und einem Katalysatorsystem, dass mindestens eine zur Übertragung von Wasserstoff auf Doppelbindungen fähige Komponente sowie Oxide der seltenen Erdmetalle oder Erdalkalimetalle oder jeweils Gemische davon enthält.

Das erfindungsgemäße Verfahren dient beispielsweise zur Herstellung von Tetrahydrogeranylaceton, einem Zwischenprodukt in der Synthese von Isophytol und Vitamin E. Durch die erfindungsgemäße Herstellung kann die Kettenverlängerung eines C₁₀-Bausteines wie Citral um drei Kohlenstoffatome auf eine besonders wirtschaftliche, einfache und atomökonomisch vorteilhafte Weise vorgenommen werden.

Im Vergleich zum erfindungsgemäßen Verfahren führt die nach dem Stand der Technik häufig praktizierte Kettenverlängerung von Hydrolinalool als C₁₀-Synthon in der Synthese von Isophytol zwangsläufig zur Emission von CO₂ (Caroll-Umlagerung unter Verwendung von Acetessigesterzwischenstufen, EP 081 16321).

Die als Ausgangsverbindungen benötigten Aldehyde der allgemeinen Formel I sowie Ketone der Formel II sind bekannte handelsübliche Verbindungen.

Mögliche Aldehyde der Formel I sind beispielsweise Prenal, Citronellal oder Citral.

Als Ketonkomponenten lassen sich beispielsweise Aceton, Methylethylketon oder Cyclohexanon verwenden. Bevorzugt verwendet man Aceton.

Das Verhältnis von Aldehyd zu Keton kann zwischen 1:1 und 1:30 betragen, bevorzugt 1:4 bis 1:15.

C₁-C₂₀-Alkyl bei den Resten R₂ und R₃ bedeutet, wenn nicht anders angegeben, einen geradkettigen oder verzweigten gesättigten Rest mit 1 bis 20 Kohlenstoffatomen, wie z.B. Methyl, Ethyl, Propyl, Butyl-, Pentyl, Hexyl, Octyl, insbesondere Methyl und Ethyl, der gegebenenfalls durch Hydroxy-, Alkoxy-, Amino- , Alkylamino- oder Carboxygruppen substituiert sein kann.

C₁-C₂₀-Alkyl bei dem Rest R₁ bedeutet, wenn nicht anders angegeben, einen geradkettigen oder verzweigten ein oder mehrfach ungesättigten Rest mit 1 bis 20 Kohlenstoffatomen, wie z.B. Propen, Buten, Hexen, Octen, Octadien, der gegebenenfalls durch Hydroxy-, Alkoxy, Amino-, Alkylamino- oder Carboxygruppen substituiert sein kann.

Cycloalkyl bedeutet, wenn nicht anders angegeben einen gesättigten, ein- oder mehrfach ungesättigten Carbocyclus mit 3 bis 7 Gerüstatomen, der gegebenenfalls ein oder mehrfach durch Hydroxy, Amino oder Carboxy substituiert sein kann, insbesondere den Cyclohexyl- oder den Cyclohexenylrest.

Das erfindungsgemäß verwendete Katalysatorsystem enthält mindestens eine, zur Übertragung von Wasserstoff auf Doppelbindungen fähige Komponente sowie Oxide der Seltenen Erdmetalle oder Erdalkalimetalle oder jeweils Gemische davon.

Als zur Übertragung von Wasserstoff auf Doppelbindungen fähige Komponenten verwendet man beispielsweise Metalle, Metalloxide oder Gemische davon, der Elemente der I. und VIII. Nebengruppe.

Als Metalle der I. Nebengruppe des Periodensystems bezeichnet man die Metalle Cu, Ag und Au. Bevorzugt verwendet man Cu.

Als Metalle der VIII. Nebengruppe des Periodensystems bezeichnet man die Metalle Eisen (Fe), Cobalt (Co), Nickel (Ni), Ruthenium (Ru), Rhodium (Rh), Palladium (Pd), Osmium (Os), Iridium (Ir) und Platin (Pt). Bevorzugt verwendet man Pd.

Die Metalle, Metalloxide oder Gemische davon, der I. bzw. VIII. Nebengruppe können jeweils einzeln oder aber als Gemisch verwendet werden. Man kann sie als solche verwenden, bevorzugt verwendet man sie jedoch auf einem inerten Trägermaterial.

Als Oxide der Erdalkalimetalle bezeichnet man erfindungsgemäß die Oxide von Beryllium (Be), Magnesium (Mg), Calcium (Ca), Strontium (Sr), Barium (Ba) oder Radon (Ra). Bevorzugt verwendet man MgO.

Als Oxide der Seltenen Erdmetalle bezeichnet man die Oxide von Lanthan (La), Cer (Ce), Praseodym (Pr), Neodym (Nd), Samarium (Sm), Europium (Eu), Gadolinium (Gd), Terbium (Tb), Dysprosium (Dy), Holmium (Ho), Erbium (Er), Thulium (Tm), Ytterbium (Yb) und Lutetium (Lu).

Bevorzugte Oxide sind Pr₂O₃, Nd₂O₃, CeO₂.

Die Katalysatoren gemäß dem erfindungsgemäßen Verfahren enthalten die Oxide der Erdalkalimetalle oder der Seltenen Erdmetalle in reiner Form oder als Gemisch der Oxide mehrerer Erdalkalimetalle oder mehrerer Seltener Erdmetalle oder als Gemisch von Erdalkaliund Seltenen Erdmetallen. Man kann die Oxide als solche verwenden, bevorzugt verwendet man sie jedoch auf einem inerten Träger.

Als inerte Träger sind beispielsweise Al₂O₃, TiO₂, SiO₂, ZrO₂, CaCO₃, BaSO₄ oder Aktivkohle zu nennen. Vorzugsweise werden Träger auf Basis von Al₂O₃ verwendet. Der Gehalt an den Oxiden auf dem inerten Träger beträgt zwischen 0,2 und 99 Gew.-%.

Die oben genannten Metalloxide der Erdalkali- bzw. der Seltenen Erdmetalle müssen weder unbedingt auf dem gleichen noch auf ein und demselben Träger aufgebracht werden. Es ist auch möglich, dass die einzelnen Komponenten zunächst jede für sich auf einen Träger aufgebracht werden und eine Vermengung der einzelnen Komponenten erst im Reaktor erfolgt.

Die erfindungsgemäße Umsetzung wird bevorzugt in flüssiger Phase durchgeführt. Sie kann sowohl kontinuierlich als auch diskontinuierlich betrieben werden. Wenn die Reaktion kontinuierlich betrieben wird, kann der Reaktionszulauf in Rieselfahrweise oder Sumpffahrweise, bevorzugt in Rieselfahrweise, über die Katalysatorschüttung gepumpt werden. Die Reaktion kann unter einem Druck von 0,1 bis 400 bar, bevorzugt 1 bis 80 bar, besonders bevorzugt 1 bis 40 bar ausgeführt werden. Die Reaktionstemperatur liegt zwischen 20 und 350°C, bevorzugt zwischen 50 bis 250°C.

Je nach Wahl der Einsatzstoffe kann die Reaktion in Gegenwart eines unter den Reaktionsbedingungen inerten Lösungsmittels durchgeführt werden. Als Lösungsmittel kommen Dialkylether, wie Di-n-butylether, aliphatischen Kohlenwasserstoffe wie Ligroin, aromatische Kohlenwasserstoffe wie Toluol und Xylol in Betracht. Bevorzugt wird die Reaktion jedoch ohne die Anwesenheit eines weiteren Lösungsmittels durchgeführt.

Das in der Reaktion entstehende Wertprodukt kann mit den üblichen Trennverfahren wie Destillation oder Kristallisation aus dem Reaktionsaustrag isoliert werden.

Mit Hilfe des erfindungsgemäßen Verfahrens ist es möglich zahlreiche höhere Ketone, beispielsweise Tetrahydrogeranylaceton mit guten Umsätzen und Selektivitäten auf einfache wirtschaftliche Weise herzustellen.

### Beispiel 1

Ein kontinuierlich betriebenes 0,3 l Reaktionsrohr wurde mit 280 g eines Katalysators der Zusammensetzung 0,9 % PdO, 10,0 % MgO und 89,1 % Al₂O₃ in Form von 4mm Strängen befüllt. In Sumpffahrweise wurden in Anwesenheit von Wasserstoff 47,5 g/h eines Gemisches aus Citronellal und Aceton im Verhältnis 1 zu 10 über das Katalysatorbett gepumpt. Die Abgasmenge betrug 2 bis 3 l/h.

Der Reaktionsaustrag wurde gaschromatographisch analysiert. Die Reaktionsbedingungen so wie die Zusammensetzung des Zulaufes und der Reaktionsausträge werden in Tabelle 1 zusammengefasst.

**Tabelle 1:**

| Versuch | Temperatur [°C] | Druck [bar] | Aceton [%] | Isopropanol [%] | Citronellal [%] | THG [%] | THGAc [%] |
|---|---|---|---|---|---|---|---|
| Zulauf | | | 76,8 | | 21,9 | | |
| 1 | 68 - 80 | 50 | 69,4 | 0,0 | 0,0 | 15,0 | 10,0 |
| 2 | 156 - 182 | 15 | 57,9 | 0,0 | 0,1 | 4,6 | 17,3 |
| THG = Tetrahydrogeraniol THGAc = Tetrahydrogeranylaceton | | | | | | | |

### Beispiel 2

Wie Beispiel 1 jedoch wurde als Reaktionszulauf ein Gemisch aus Citral (E,Z-Isomerengemisch) und Aceton im Molverhältnis von 1 zu 10 verwendet.

Die Reaktionsbedingungen so wie die Zusammensetzung des Zulaufes und der Reaktionsausträge werden in Tabelle 2 zusammengefasst.

**Tabelle 2:**

| Versuch | Temperatur [°C] | Druck [bar] | Aceton [%] | Isopropanol [%] | Citral [%] | THG [%] | THGAc [%] |
|---|---|---|---|---|---|---|---|
| Zulauf | | | 76,3 | | 23,0 | | |
| 1 | 156 - 181 | 15 | 61,3 | 0,0 | 0,1 | 1,1 | 21,5 |

### Beispiel 3

Wie Beispiel 1 jedoch wurde ein Katalysator mit der Zusammensetzung 0,5 % PdO, 5,0 % Pr₂O₃ und 94,5 % Al₂O₃ in Form von 4mm Strängen verwendet.

Die Reaktionsbedingungen so wie die Zusammensetzung des Zulaufes und der Reaktionsausträge werden in Tabelle 3 zusammengefasst.

**Tabelle 3:**

| Versuch | Temperatur [°C] | Druck [bar] | Aceton [%] | Isopropanol [%] | Citronellal [%] | THG [%] | THGAc [%] |
|---|---|---|---|---|---|---|---|
| Zulauf | | | 76,8 | | 21,9 | | |
| 1 | 113 - 131 | 15 | 48,9 | 12,4 | 0,0 | 1,4 | 20,5 |

### Beispiel 4

Wie Beispiel 3 jedoch wurde als Reaktionszulauf ein Gemisch aus Citral (E,Z-Isomerengemisch) und Aceton im Molverhältnis von 1 zu 10 verwendet.

Die Reaktionsbedingungen so wie die Zusammensetzung des Zulaufes und der Reaktionsausträge werden in Tabelle 4 zusammengefasst.

**Tabelle 4:**

| Versuch | Temperatur [°C] | Druck [bar] | Aceton [%] | Isopropanol [%] | Citral [%] | THG [%] | THGAc [%] |
|---|---|---|---|---|---|---|---|
| Zulauf | | | 76,3 | | 23,0 | | |
| 1 | 96 - 116 | 15 | 68,3 | 0,0 | 0,03 | 0,9 | 22,1 |

### Beispiel 5

Wie Beispiel 4 jedoch wurde ein Katalysator mit der Zusammensetzung 0,5 % PdO, 19,7 % CaO und 79,8 % Al₂O₃ verwendet.

Die Reaktionsbedingungen so wie die Zusammensetzung des Zulaufes und der Reaktionsausträge werden in Tabelle 5 zusammengefasst.

**Tabelle 5:**

| Versuch | Temperatur [°C] | Druck [bar] | Aceton [%] | Isopropanol [%] | Citral [%] | THG [%] | THGAc [%] |
|---|---|---|---|---|---|---|---|
| Zulauf | | | 76,3 | | 23,0 | | |
| 1 | 113 - 133 | 15 | 58,3 | 0,0 | 0,03 | 3,2 | 17,6 |

## Patentansprüche

1. Verfahren zur Herstellung von höheren Ketonen durch Umsetzung eines Aldehyds der allgemeinen Formel I 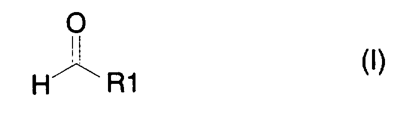 wobei
R¹ einen gegebenenfalls substituierten geradkettigen oder verzweigten ein- oder mehrfach ungesättigten C₁-C₂₀-Alkylrest oder einen gegebenenfalls substituierten C₃-C₇-Cycloalkenylrest bedeutet
mit einem Keton der allgemeinen Formel II 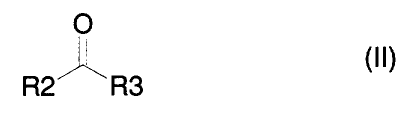 wobei
R², R³ einen gegebenenfalls substituierten geradkettigen oder verzweigten gesättigten oder ungesättigten C₁-C₂₀-Alkylrest oder einen gegebenenfalls substituierten C₃-C₇-Cycloalkyl-, oder Cycloalkenylrest bedeuten oder R² und R³ zusammen einen 3-7-gliedrigen, gegebenenfalls substituierten Carbocyclus bedeuten in Gegenwart von Wasserstoff und einem Katalysatorsystem, dass mindestens eine zur Übertragung von Wasserstoff auf Doppelbindungen fähige Komponente sowie Oxide der Seltenen Erdmetalle oder Erdalkalimetalle oder jeweils Gemische davon enthält.

2. Verfahren gemäß Anspruch 1, **dadurch gekennzeichnet, dass** der Aldehyd der allgemeinen Formel I, ausgewählt ist aus der Gruppe Citronellal, Citral oder Prenal.

3. Verfahren gemäß einem der Ansprüche 1 oder 2, **dadurch gekennzeichnet, dass** das Keton der allgemeinen Formel II ausgewählt ist aus der Gruppe Aceton, Methylethylketon oder Cyclohexanon.

4. Verfahren gemäß einem der Ansprüche 1 bis 3, **dadurch gekennzeichnet, dass** die Umsetzung in Gegenwart eines Katalysatorsystems ausgewählt aus der Gruppe Magnesiumoxid, oder Praseodymoxid, Neodymoxid oder Ceroxid in Gegenwart einer zur auf Doppelbindungen wasserstoffübertragenden fähigen Komponente durchgeführt wird.

5. Verfahren gemäß einem der Ansprüche 1 bis 4, **dadurch gekennzeichnet, dass** als auf Doppelbindungen wasserstoffübertragende fähige Komponente Palladiumoxid eingesetzt wird.

6. Verfahren gemäß einem der Ansprüche 1 bis 5, **dadurch gekennzeichnet, dass** die Umsetzung in Gegenwart eines Katalysatorsystems durchgeführt wird, dass ein Gemisch aus Magnesiumoxid und Palladiumoxid enthält.

7. Verfahren gemäß einem der Ansprüche 1 bis 6, **dadurch gekennzeichnet, dass** der Gehalt an dem Oxid der Erdalkalimetalle oder der Seltenen Erden oder Gemischen davon auf dem inerten Träger 0,2 bis 99 Gew.% beträgt.

8. Verfahren gemäß einem der Ansprüche 1 bis 7, **dadurch gekennzeichnet, dass** als inerter Träger Al₂O₃, TiO₂, SiO₂, ZrO₂, CaCO₃, BaSO₄ oder Aktivkohle verwendet wird.

9. Verfahren gemäß einem der Ansprüche 1 bis 8, **dadurch gekennzeichnet, dass** man die Umsetzung des Aldehyds der Formel I mit dem Keton der Formel II bei Temperaturen von 20 bis 350°C und einem Druck von 0,1 bis 400 bar durchführt.

10. Verfahren gemäß einem der Ansprüche 1 bis 9, **dadurch gekennzeichnet, dass** das Verhältnis Aldehyd zu Keton zwischen 1:1 und 1:30 liegt.

11. Verfahren gemäß einem der Ansprüche 1 bis 10 zur Herstellung von Tetrahydrogeranylaceton.

12. Verwendung von Tetrahydrogeranylaceton gemäß Anspruch 11 als Zwischenprodukt zur Herstellung von Isophytol.

13. Katalysatorsystem mit sowohl kondensierenden als auch hydrierenden Eigenschaften, enthaltend mindestens einer zur Übertragung von Wasserstoff auf Doppelbindungen befähigte Komponente, sowie ein Oxid der Erdalkalimetalle, der Seltenen Erden oder ein Gemisch davon.
